# EUROPEAN PATENT SPECIFICATION

(11) **EP 0 654 259 B1**
(45) Date of publication and mention of the grant of the patent: **23.08.2000**
(21) Application number: 94118210.7
(22) Date of filing: 18.11.1994
(51) Int. Cl.: A61K 7/06, A61K 7/50

(54) **Hair conditioner composition**
Mittel zur Haarpflege
Composition de conditionnement de cheveux

(30) Priority: 19.11.1993 JP 29071793
(43) Date of publication of application: 24.05.1995
(73) Proprietor: Kao Corporation, Chuo-Ku Tokyo 103 (JP)
(72) Inventor: Morita, Kouzi, Mitaka-shi, Tokyo (JP); Yahagi, Kazuyuki, Sumida-ku, Tokyo (JP)
(74) Representative: Hansen, Bernd, Dr. Dipl.-Chem.

(56) References cited:
- EP-A- 0 300 379
- EP-A- 0 538 762
- EP-A- 0 555 690
- WO-A-94/02111
- CHEMICAL ABSTRACTS, vol. 97, no. 10, September 1982, Columbus, Ohio, US; abstract no. 78683, 'hair rinses containong quaternary ammonium salts and fatty esters' & JP-A-57 053 402 (KAO SOAP)
- S.T.N., File Supplier, Karlsruhe, DE, File Chemical Abstacts, vol 120, n 143674, * abstract * & JP-A-05 286 831 (KAO CORP.)
- S.T.N., File Supplier, Karlsruhe, DE, File Chemical Abstracts, vol 118, n 27261 * abstract * & JP-A-04 230 615 (KAO CORP)
- PATENT ABSTRACTS OF JAPAN vol. 6, no. 131 (C-114) (1009) & JP-A-57 056 412 (RAION K.K.)

## Description

This invention relates to a hair conditioner composition and more particularly a hair conditioner composition showing a pearl-like appearance (a pearly luster), having an UV ray-screening effect, imparting a natural gloss to hair, and preventing hair from being disheveled during sleep.

To impart a pearl-like appearance, fish scale guanine or an inorganic mineral, such as mica-based synthetic pearl or titanium dioxide, is contained in some conventional hair cosmetic compositions. Also, to impart gloss to hair, a liquid oil or silicone is contained in some conventional hair cosmetic compositions.

However, the inorganic minerals sometimes which are added in the hair cosmetic composition to impart thereto a pearl-like appearance sometimes cause a squeaky feel or a stiff feel to hair. On the other hand, a hair cosmetic composition containing the liquid oil or silicone can impart an oily gloss to hair but it hardly produces a natural gloss.

It has therefore been demanded to develop a hair conditioner composition which has a beautiful pearl-like appearance and is capable of imparting a natural gloss to hair.

EP-A-0 538 762 describes a hair cosmetic comprising a cationic surfactant, a fat and/or an oil, and an alkyl saccharide surfactant having a specified formula. The weight ratio of the cationic surfactant and the alkyl saccharide surfactant is indicated to be from 1 to 20. However, no weight ratio is indicated with respect to the fat and oil and the cationic surfactant.

JP-57-56412 describes a hair cosmetic comprising a quaternary ammonium salt containing a C₁₆₋₁₈ alkyl group, a quaternary ammonium salt containing a C₂₀₋₂₂ alkyl group and a fatty acid as essential components, and capable of imparting improved luster and smoothness to the hair. Having regard to the examples of the fat and oil to be used, a plurality of different compounds such as higher alcohols, hydrocarbons, lanolin derivatives, higher fatty acid esters, higher fatty acids and long chain amide amines and vegetable and animal fats and oils are cited. It is particularly preferred to use monoglycerides originated from saturated or unsaturated, straight chain or branched chain fatty acids having 12 to 24 carbon atoms, and higher alcohols having straight chain or branched alkyl or alkenyl groups having 12 to 26 carbon atoms.

Chemical Abstracts, vol. 97 (1982): 97: 78683x describes hair rinses containing quaternary ammonium salts and fatty acid esters. Stearic and isostearic acid are used to prepare the fatty acid ester.

JP-A-0 52 86 831 describes hair conditioners containing ammonium- or amine- form cationic surfactants and higher fatty acids in a weight ratio of the surfactant to the acids of 10:1 to 1:10. According to a particular example the fatty acid is isostearic acid which is used in the identical amount, based on percent by weight, as the cationic surfactant.

As a result of extensive study, the present inventors found that a hair conditioner composition containing a specific fatty acid and a cationic surfactant at a specific ratio shows a beautiful pearl-like appearance, imparts a natural gloss to hair, have an excellent UV ray-screening effect, and prevent hair from being disheveled because of sleep.

Namely, the present invention provides a hair conditioner composition comprising:
(A) a fatty acid having a straight-chain acyl group containing 18 to 28 carbon atoms, and
(B) a cationic surfactant
   wherein a weight ratio of component (A) to component (B) is from 1.5/1 to 4/1.

The present invention also provides a method for imparting gloss to hair which comprises applying to hair the hair conditioner composition.

Examples of the fatty acid as component (A) include stearic acid, nonadecanoic acid, arachic acid, behenic acid, lignoceric acid, cerotic acid, heptacosanoic acid, and montanic acid. Among them, behenic acid is particularly preferred since it is easy to be formulated into cosmetics, it is easily available from commercial products and it gives an excellent pearl-like appearance. These fatty acids are industrially available.

From the standpoint of viscosity suitable for use, component (A) is preferably used in an amount of from 1.0 to 15.0 % by weight, particularly from 3.0 to 10.0 % by weight, based on the total composition.

The cationic surfactant as component (B) includes quaternary ammonium salts represented by formula (1) or (2): 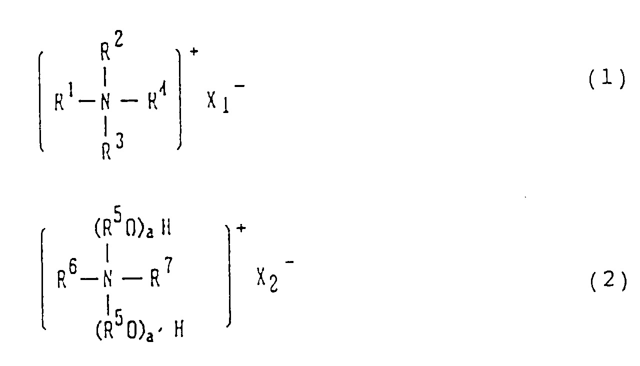 wherein at least one of R¹, R², R³, and R⁴ represents an alkyl or alkenyl group having 8 to 28 carbon atoms in total, which may be substituted by a substituent selected from an alkoxy, alkenyloxy, alkanoylamino and alkenoylamino groups, and the other(s) represent a benzyl group or an alkyl or hydroxyalkyl group having 1 to 5 carbon atoms; R⁵ represents an alkylene group having 2 or 3 carbon atoms; X₁⁻ and X₂⁻ each represents a halogen ion or an organic anion; a and a' each represents an integer of 1 to 20; and at least one of R⁶ and R⁷ represents an alkyl or alkenyl group having from 8 to 28 carbon atoms in total, which may be substituted by a substituent selected from an alkoxy, alkenyloxy, alkanoylamino and alkenoylamino groups, and the other represents a benzyl group or an alkyl or hydroxyalkyl group having 1 to 5 carbon atoms.

Among these quaternary ammonium salts, ones represented by formula (1) are preferred, and of the quaternary ammonium salts represented by formula (1), ones represented by following formula (3), (4) or (5) are particularly preferred. 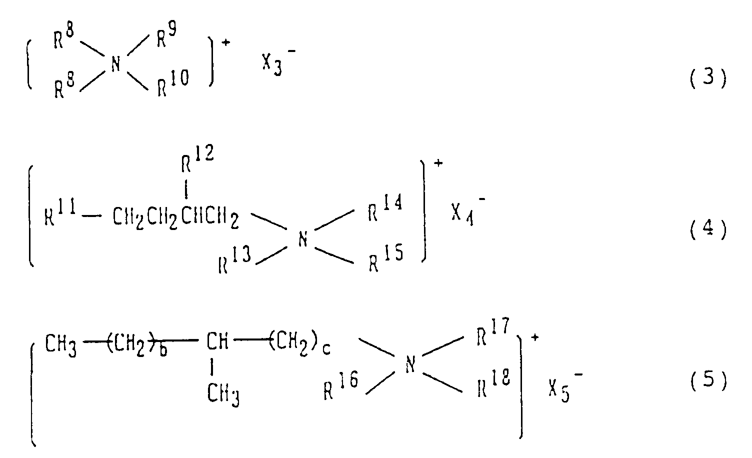 wherein R⁸ represents a mixture of (a) a branched alkyl group represented by formula: 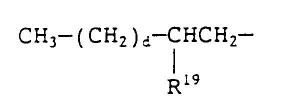
wherein R¹⁹ represents a methyl group or an ethyl group; and d represents an integer to give a total carbon atom number to the branched alkyl group (a) of 8 to 16,
and (b) a straight-chain alkyl group represented by formula:

CH₃―(CH₂)ₑ―

wherein e represents an integer of 7 to 15,
the mixture having a branching ratio (a)/((a)+(b)) of from 10 to 100 %; R⁹ and R¹⁰ each represents a benzyl group or an alkyl or hydroxyalkyl group having 1 to 3 carbon atoms; R¹¹ and R¹² each represents an alkyl group having 2 to 12 carbon atoms; R¹³ represents a group of formula: 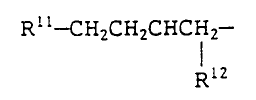 or an alkyl group having 1 to 3 carbon atoms; R¹⁴ and R¹⁵ each represents a benzyl group or an alkyl or hydroxyalkyl group having 1 to 3 carbon atoms; R¹⁶ represents a group of formula: 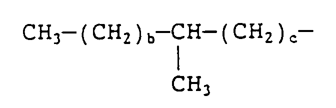
wherein b represents an integer of 2 to 14; and c represents an integer of 3 to 11; provided that the sum of b and c is from 9 to 21,
or an alkyl group having 1 to 3 carbon atoms; R¹⁷ and R¹⁸ each represents a benzyl group or an alkyl or hydroxyalkyl group having 1 to 3 carbon atoms; and X₃⁻, X₄⁻, and X₅⁻ each represents a halogen ion or an organic anion.

The branched quaternary ammonium salts represented by formula (3) may be synthesized by using, for example, an oxo alcohol having 8 to 16 carbon atoms. Examples of the compounds of formula (3) include dialkyldimethylammonium salts, dialkylmethylhydroxyethylammonium salts and dialkylmethylbenzylammonium salts, the alkyl moiety of which is derived from the oxo alcohol.

The branching ratio (a)/((a)+(b)) of R⁸ in formula (3) is usually from 10 to 100 % and preferably from 10 to 50 %. The total carbon atom number of the alkyl group represented by R⁸ is from 8 to 16. It is preferable that the mixed alkyl group has a certain distribution of carbon atom number, especially a distribution shown below:
- C₈₋₁₁:: 5 % or less
- C₁₂:: 10 to 35 %
- C₁₃:: 15 to 40 %
- C₁₄:: 20 to 45 %
- C₁₅:: 5 to 30 %
- C₁₆:: 5 % or less

Examples of the compounds of formula (3) include a dialkyldimethylammonium chloride wherein the alkyl group has from 8 to 16 carbon atoms and a branching ratio of from 10 to 50 %.

The branched quaternary ammonium salts of formula (4) are generally synthesized from a Guerbet alcohol represented by formula (6): 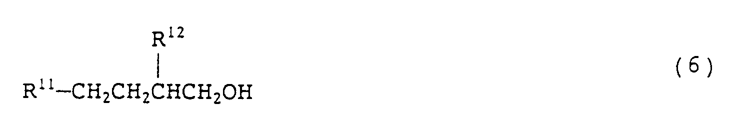 wherein R¹¹ and R¹² are as defined above.

Preferred examples of the quaternary ammonium salt of formula (4) include alkyltrimethylammonium salts, alkyldimethylbenzylammonium salts, dialkyldimethylammonium salts, dialkylmethylhydroxyethylammonium salts and dialkylmethylbenzylammonium salts, the alkyl moiety of which is derived from a Guerbet alcohol. Among them, 2-decyltetradecyltrimethylammonium chloride, 2-dodecylhexadecyltrimethylammonium chloride, di-2-hexyldecyldimethylammonium chloride, and di-2-octyldodecyldimethylammonium chloride are preferred.

Examples of the methyl-branched quaternary ammonium salt of formula (5) include those in which the sum of b and c is 15.

The quaternary ammonium salt as component (B) further includes quaternary ammonium salts represented by formula (7): 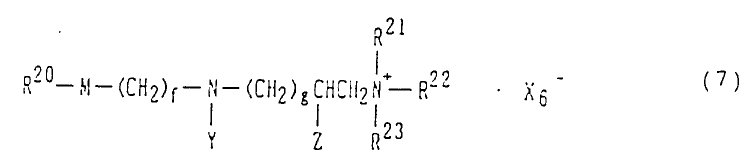 wherein R²⁰ represents a straight-chain or branched alkyl or alkenyl group having 7 to 21 carbon atoms; R²¹, R²², and R²³ each represents an alkyl or hydroxyalkyl group having 1 to 4 carbon atoms; M represents -CONG- (wherein G represents a hydrogen atom or an alkyl or hydroxyalkyl group having 1 to 3 carbon atoms, provided that the carbonyl group of which is bonded to R²⁰), -O- or -COO- (provided that the carbonyl group of which is bonded to R²⁰); X₆⁻ represents a halogen ion or an organic anion; f represents 2 or 3; g represents 0 or an integer of 1 to 5; Z represents a hydrogen atom or a hydroxyl group; and Y represents a hydrogen atom, an alkyl or hydroxyalkyl group having 1 to 3 carbon atoms, or a group represented by formula (8): 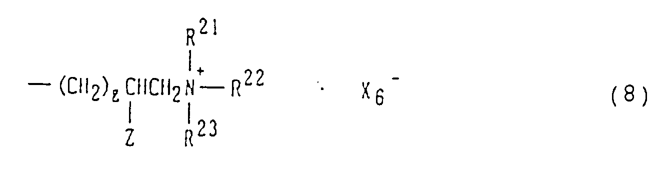 wherein R²¹, R²², R²³, X₆⁻, Z, and g are as defined above; provided that G and Y do not represent an alkyl or hydroxyalkyl group having 1 to 3 carbon atoms simultaneously and that when g is 0, 2, 3, 4 or 5, Z represents a hydrogen atom.

Of the cationic surfactants of formula (7), those represented by formula (9) are preferred: 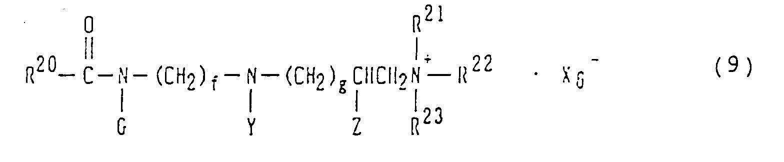 wherein R²⁰, R²¹, R²², R²³, X₆⁻, G, Y, Z, f, and g are as defined above.

In particular, those represented by formula (10) are preferred: 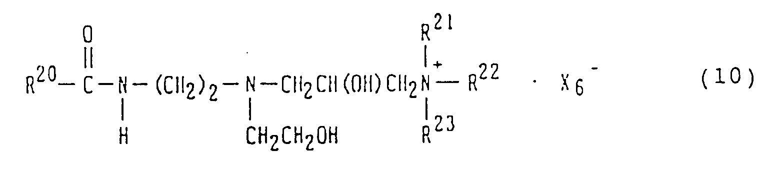 wherein R²⁰, R²¹, R²², R²³, and X₆⁻ are as defined above.

The quaternary ammonium salts of formula (7) can be prepared by known methods, for example, the method described in JP-A-5-155835 (the term "JP-A" as used herein means an "unexamined published Japanese patent application"). For instance, the compound of formula (10) can he obtained by reacting a corresponding fatty acid with aminoethyl-ethanolamine to form an imidazoline derivative, which is further treated with an alkali and then reacted with a corresponding ammonium compound as shown in the following reaction scheme: 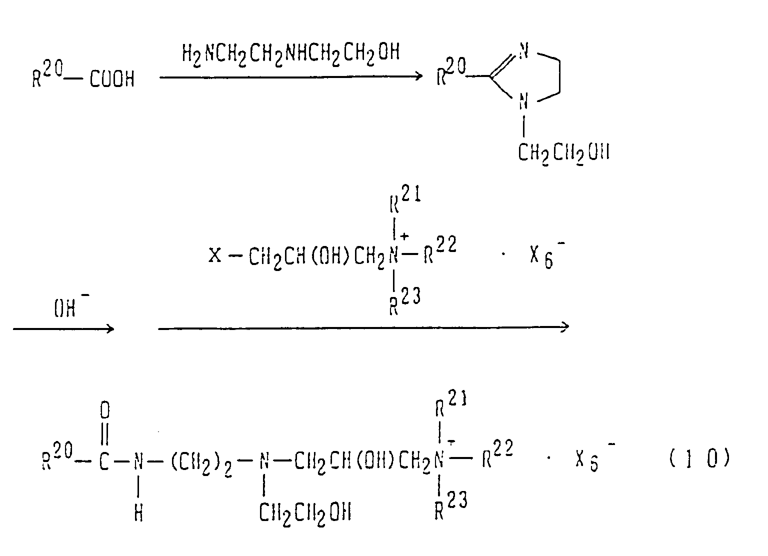 wherein R²⁰, R²¹, R²², R²³, and X₆⁻ are as defined above; and X represents a halogen atom or an organic anionic group.

It is preferred that the product as obtained by the above method is desalted by, for example, electrodialysis to improve the dissolving characteristics or the viscosity characteristics.

The quaternary ammonium salt as component (B) furthermore includes asymmetric quaternary ammonium salts represented by formula (11): 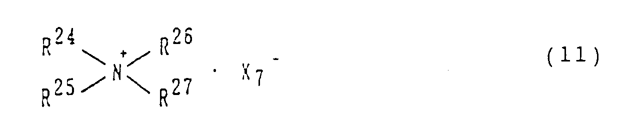 wherein R²⁴ represents a branched alkyl group having 8 to 28 carbon atoms in total; R²⁵ represents a straight-chain alkyl or alkenyl group having 8 to 22 carbon atoms; R²⁶ and R²⁷ each represent a hydrogen atom or an alkyl group having 1 to 4 carbon atoms; and X₇⁻ represents a halogen ion or an organic anion; provided that R²⁶ and R²⁷ do not represent a hydrogen atom simultaneously.

The alkyl group represented by R²⁴ in formula (11) is derived from a Guerbet alcohol or an oxo alcohol as described above. Examples of the alkyl group as R²⁴ include 2-(3-methylhexyl)-7-methyl-1-decyl, 2-(1-methyl-3,3-dimethylbutyl)-5-methyl-7,7-dimethyloctyl, and 2-hexyl-1-decyl groups.

The terminology "Guerbet alcohol" as used herein generally means an alcohol represented by formula (6), and the terminology "oxo alcohol" as used herein generally means an alcohol obtained from an α-olefin by an oxo process and derivatives thereof. Examples of the oxo alcohol include alcohols represented by formulae (12) and (13): 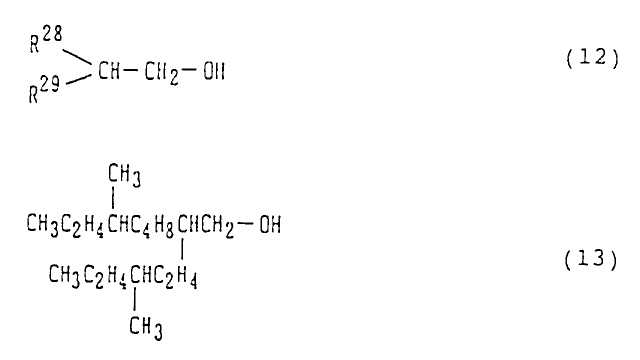 wherein R²⁸ represents an alkyl group having 1 to 5 carbon atoms; and R²⁹ represents an alkyl group having 5 to 10 carbon atoms; provided that the total carbon atom number in R²⁸ and R²⁹ is 10 or 11.

Specific examples of these alcohols are Fine Oxo Alcohol 140, 1600, 180, 180N, 1800, 2000 or 2600, produced by Nissan Chemical Industries, Ltd.; Diadol 18G, produced by Mitsubishi Chemical Industries Ltd.; Dobanol 23-1, produced by Mitsubishi Petrochemical Co., Ltd; EXXAL 18 and EXXAL 20, produced by Exxon Kagaku K.K.; Emasol 871, produced by Emery Industries Inc.; and isostearyl alcohol obtained by methyl esterifying Evasol 871, followed by reduction.

Preferred examples of the asymmetric quaternary ammonium salts of formula (11) include N-2-(3-methylhexyl)-7-methyl-1-decyl-N-dodecyl-N,N-dimethylammonium chloride, N-2-(3-methylhexyl)-7-methyl-1-decyl-N-octyl-N,N-dimethylammonium chloride, N-2-hexyl-1-decyl-N-dodecyl-N,N-dimethylammonium chloride, and compounds represented by formula (14): 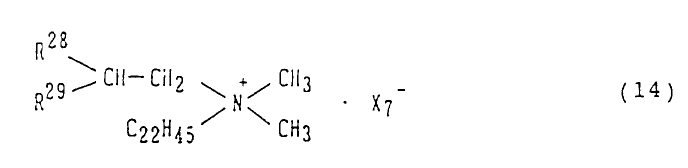 wherein R²⁸, R²⁹, and X₇⁻ are as defined above.

The halogen ion or organic anion represented by X₁⁻, X₂⁻, X₃⁻, X₄⁻, X₅⁻, X₆⁻, or X₇⁻ includes a chloride ion, a bromide ion, and an iodide ion, or a methosulfate ion, an ethosulfate ion, a methophosphate ion, and an ethophosphate ion.

The above-mentioned cationic surfactants may be used either singly or in combination of two or more thereof. From the standpoint of the viscosity suitable for use, component (B) is preferably used in an amount of from 0.25 to 7.5 % by weight, more preferably from 0.75 to 5 % by weight, based on the total composition.

A weight ratio of component (A) to (B) in the hair conditioner composition of the present invention is from 1.5/1 to 4/1, preferably from 2.5/1 to 3.5/1. If it is less than 1.5/1, a satisfactory pearly luster cannot be obtained. If it exceeds 4/1, the whole system has poor stability.

A higher alcohol may be contained in the hair conditioner composition of the present invention. However, it is important that the hair conditioner composition of the present invention should not contain higher alcohol(s) in an amount exceeding 1/3 by weight of component (A). The terminology "higher alcohol" as used herein means mono- or polyhydric alcohol whose hydrocarbon group has 7 or more carbon atoms. If the content of such higher alcohols exceeds 1/3 the weight of component (A), the pearly luster of the hair conditioner composition tends to be lost.

The hair conditioner compositions of the present invention preferably have a pH value of from 2 to 7, more preferably from 3 to 6. If the pH value is less than 2, the composition is too irritative to the skin, thus such is not preferred in view of safety. If it exceeds 7, the composition hardly shows a pearl-like appearance. The pH can be adjusted with known acidic or alkaline chemicals generally employed in hair cosmetics.

If desired, the hair conditioner compositions of the present invention may contain other components usually added to hair cosmetics so long as the effects of the present invention are not impaired. Such optional components include additives for improving feel to the touch, such as squalene, lanolin, dimethylpolysiloxane, high-molecular weight polysiloxane, methylphenylpolysiloxane, water-soluble polyether-modified silicone, amino-modified silicone, carboxyl-modified silicone, perfluoropolyethers, and cationic polymers described in JP-A-58-53996 and JP-A-1-117821; humectants, such as propylene glycol, glycerin, and sorbitol; viscosity modifiers, such as methyl cellulose, carboxyvinyl polymers, hydroxyethyl cellulose, polyoxyethylene glycol distearate, and ethanol; pearling agents; perfumes; colorants; UV absorbents; antioxidants; bactericides, such as Triclosan and Trichlocarban; antiinflammatory agents, such as potassium glycyrrhizinate and tocopherol acetate; anti-dandruff agents, such as zinc pyrithione and Octopirox; antiseptics, such as methylparaben and butylparaben; and other additives as described in Encyclopedia of Shampoo Ingredients, Micelle Press (1985).

The hair conditioner composition of the present invention can be prepared by uniformly mixing the above-mentioned components in a conventional manner. The hair conditioner composition according to the present invention may be formulated into a hair treatment, a hair rinse, a hair conditioning foam, a hair styling preparations, a permanent wave setting lotion, a hair dye, a coloring rinses and a hair cream.

The hair conditioner composition of the present invention shows a beautiful pearl-like appearance, imparts a natural gloss to hair, exhibits an UV ray-screening effect, and prevents hair from being disheveled during sleep.

The present invention will now be illustrated in greater detail with reference to Examples, but it should be understood that the present invention is not construed as being limited thereto. All the percents are given by weight except where noted.

### EXAMPLE 1

Hair treatment compositions of the composition shown in Table 1 were prepared in a conventional manner. The pearl-like appearance of the composition and the effects of the composition in glossing, UV-screening, and preventing hair from being disheveled during sleep were evaluated in the following manner. The results are shown in Table 1.

### (1) Pearl-like appearance:

The pearl-like appearance of the composition was observed with the naked eye and rated in accordance with the following criteria.

### (Evaluation Criteria)

- A:: Excellent pearly luster
- B:: Pearly luster
- C:: Equivocal
- D:: None

### (2) Gloss:

About 20 g of cold-permed hair of a Japanese woman (about 15 to 20 cm in length) was tied up in a bundle. After shampooing, 2 g of the hair treatment composition was uniformly applied to the hair, rinsed with running warm water at about 40°C, and dried with a hair drier. The gloss of the thus treated hair was evaluated in accordance with the following criteria in comparison with the hair which was subjected to shampooing but not subjected to treatment with the hair treatment composition.

### (Evaluation Criteria)

- A:: Very glossy
- B:: Glossy
- C:: Equivocal
- D:: None

### (3) UV ray-screening effect:

About 20 g of cold-permed hair of a Japanese woman (about 15 to 20 cm in length) was tied up in a bundle. After shampooing, 1 g of the hair treatment composition was uniformly applied to the hair, rinsed with running warm water at about 40°C, and dried with a hair drier. The thus treated hair was exposed to UV light having a wavelength of 310 nm and an intensity of 0.7 mW/cm² for 100 hours by means of a solar simulator. The change of the color of the hair was observed with the naked eye and rated in accordance with the following criteria.
- A:: No change
- B:: Almost no change
- C:: Slight change
- D:: Noticeable change

### (4) Prevention of Hair from being disheveled during sleep:

Ten female panel members shampooed in the evening, applied about 12 g of each of the hair treatment composition shown in Table 1 all over their hair, rinsed the treatment composition away in a usual manner, and took usual actions before their sleep. On the next morning, the state of their hair in terms of degree of the dishevelment was evaluated in comparison with that of the hair treated with Comparative Product 1 in accordance with the following criteria.

### (Evaluation Criteria)

As compared with Comparative Product 1, a composition which gave a better result (less disheveling of hair) was rated 2, a composition which gave a slight better result was rated 1, a composition which gave an equivocal result was rated 0, a composition which gave a slight worse result was rated -1, and a composition which gave a worse result was rated -2. The average of the total points of each sample thus rated was graded as follows.
- A:: 1.0 or more
- B:: 0.5 or more but less than 1.0
- C:: -0.5 or more but less than 0.5
- D:: -1.0 or more but less than -0.5
- E:: less than -1.0

**TABLE 1**

| | Product of the Invention | | | Comparative Product | | | |
|---|---|---|---|---|---|---|---|
| Composition (% by weight) | 1 | 2 | 3 | 1 | 2 | 3 | 4 |
| Stearyltrimethylammoniun chloride | 2 | 2 | 1 | 2 | 2 | 1 | 2 |
| Dialkyldimethylammonium chloride^{*1} | - | 1 | 1 | - | 1 | 1 | - |
| N-(2-Decyl)tetradecyl-N,N,N-trimethylammonium chloride^{*2} | - | - | 1 | - | - | 1 | - |
| Behenic acid | 8 | 6 | 9 | 4 | - | - | 2 |
| Stearic acid | - | - | - | - | - | - | - |
| Cetyl alcohol | - | - | - | 2 | 6 | 9 | - |
| Liquid lanolin | 1 | 1 | 1 | 1 | 1 | 1 | 1 |
| Vaseline | 1 | 1 | 1 | 1 | 1 | 1 | 1 |
| Liquid paraffin | 1 | 1 | 1 | 1 | 1 | 1 | 1 |
| Sodium hydroxide to | | | | | | | |
| adjust to | pH3 | pH4 | - | pH4 | pH4 | pH4 | pH4 |
| Triethanolamine to adjust to | - | - | pH4 | - | - | - | - |
| Purified water | balance | balance | balance | balance | balance | balance | balance |

| Results of Evaluation: | | | | | | | |
|---|---|---|---|---|---|---|---|
| Pearl-like appearance | A | B | A | C | D | D | D |
| Hair gloss | A | B | A | C | C | C | D |
| UV ray-screening effect | A | B | A | C | D | D | D |
| Prevention of hair from being disheveled during sleep | A | A | A | C | D | D | C |

| | | | | | | | |
|---|---|---|---|---|---|---|---|
| Note: *1: Branched quaternary ammonium salt (branching ratio (degree of branching): 20 %), derived from a commercially available C₁₂₋₁₅ oxo alcohol (a mixture of Dovanol 23 and Dovanol 45, produced by Mitsubishi petrochemical Co., Ltd.) | | | | | | | |
| *2: Synthesized from a Guerbet alcohol (Ejcol 240A, produced by New Japan Chemical Co., Ltd.) | | | | | | | |

As is apparent from the results shown in Table 1, all of Products of the Invention are superior to Comparative Products in terms of the pearl-like appearance, property of imparting a natural gloss to hair, UV ray-screening effect, and property of preventing hair from being disheveled during sleep.

### EXAMPLE 2

A hair rinse having the following composition was prepared in a conventional manner.

| Component | Amount (% by weight) |
|---|---|
| Distearyldimethylammonium chloride | 1.0 |
| Cetyltrimethylammonium chloride | 0.5 |
| Behenic acid | 4.0 |
| Stearic acid | 1.0 |
| Propylene glycol | 5.0 |
| Isopropyl myristate | 2.0 |
| Methylparaben | 0.1 |
| Perfume | 0.3 |
| Ion-exchanged water | Balance |

### EXAMPLE 3

A hair conditioning foam having the following composition was prepared in a conventional manner.

| Component | Amount (% by weight) |
|---|---|
| Stearyltrimethylammonium chloride | 0.2 |
| N-(2-Dodecyl)hexadecyl-N,N,N-trimethylammonium chloride | 0.1 |
| Behenic acid | 1.0 |
| Glycerin | 2.0 |
| Ethanol | 5.0 |
| Polyoxyethylene(5) lauryl ether | 0.3 |
| Liquid paraffin | 0.5 |
| Methylpolysiloxane (200 cs) | 0.5 |
| Perfume | 0.1 |
| Propellant (LPG) | 10.0 |
| Ion-exchanged water | Balance |

### EXAMPLE 4

A styling preparation for hair setting with a drier having the following composition was prepared in a conventional manner.

| Component | Amount (% by weight) |
|---|---|
| Cetyltrimethylammonium chloride | 0.3 |
| Behenic acid | 1.0 |
| Polymethacrylic ester | 0.2 |
| Polyethylene glycol 30000 | 0.1 |
| Ethanol | 20.0 |
| Perfume | 0.2 |
| Ion-exchanged water | Balance |

The hair conditioner compositions prepared in Examples 2 to 4 all showed a beautiful pearl-like appearance, imparted a natural gloss to hair, exhibited an excellent UV ray-screening effect, and prevented hair from being disheveled during sleep.

## Claims

1. A hair conditioner composition comprising:
(A) a fatty acid having a straight-chain acyl group containing 18 to 28 carbon atoms, and
(B) a cationic surfactant
wherein a weight ratio of component (A) to component (B) is from 1.5/1 to 4/1.

2. The hair conditioner composition of claim 1, wherein said composition has a pH of from 2 and 7.

3. The hair conditioner composition of claims 1 or 2 wherein component (A) is present in an amount of from 1.0 to 15.0 % by weight and component (B) is present in an amount of from 0.25 to 7.5 % by weight.

4. the hair conditioner composition of any one of claims 1 to 3 wherein said component (A) is behenic acid.

5. The hair conditioner composition of any one of claims 1 to 4 wherein component (B) is at least one quaternary ammonium salt selected from the group consisting of those represented by formulae (1), (2), (7), and (11): 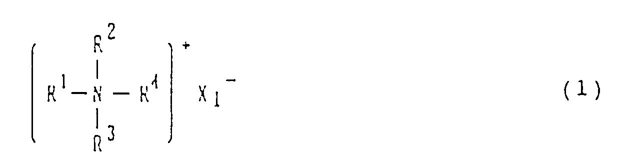 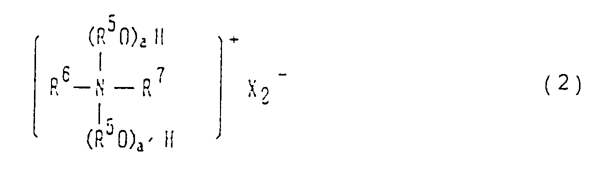 wherein at least one of R¹, R², R³, and R⁴ represents an alkyl or alkenyl group having 8 to 28 carbon atoms in total, which may be substituted by a substituent selected from an alkoxy, alkenyloxy, alkanoylamino and alkenoylamino groups, and the other(s) represent a benzyl group or an alkyl or hydroxyalkyl group having 1 to 5 carbon atoms; R⁵ represents an alkylene group having 2 or 3 carbon atoms; X₁⁻ and X₂⁻ each represents a halogen ion or an organic anion; a and a' each represents an integer of 1 to 20; and at least one of R⁶ and R⁷ represents an alkyl or alkenyl group having from 8 to 28 carbon atoms in total, which may be substituted by a substituent selected from an alkoxy, alkenyloxy, alkanoylamino and alkenoylamino groups, and the other represents a benzyl group or an alkyl or hydroxyalkyl group having 1 to 5 carbon atoms; 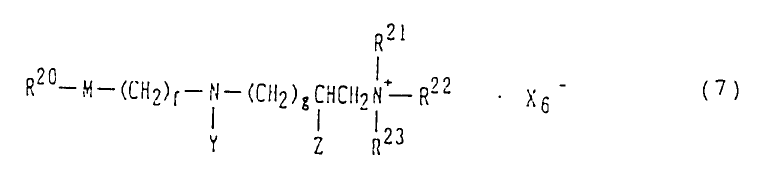 wherein R²⁰ represents a straight-chain or branched alkyl or alkenyl group having 7 to 21 carbon atoms; R²¹, R²², and R²³ each represents an alkyl or hydroxyalkyl group having 1 to 4 carbon atoms; M represents -CONG- (wherein G represents a hydrogen atom or an alkyl or hydroxyalkyl group having 1 to 3 carbon atoms, provided that the carbonyl group of which is bonded to R²⁰), -O- or -COO- (provided that the carbonyl group of which is bonded to R²⁰); X₆⁻ represents a halogen ion or an organic anion; f represents 2 or 3; g represents 0 or an integer of 1 to 5; Z represents a hydrogen atom or a hydroxyl group; and Y represents a hydrogen atom, an alkyl or hydroxyalkyl group having 1 to 3 carbon atoms, or a group represented by formula (8): 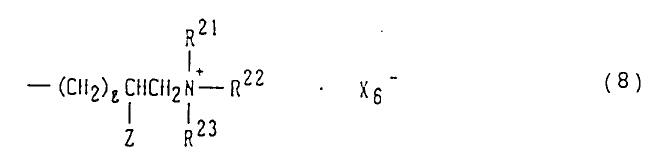 wherein R²¹, R²², R²³, X₆⁻, Z, and g are as defined above; provided that G and Y do not represent an alkyl or hydroxyalkyl group having 1 to 3 carbon atoms simultaneously and that when g is 0, 2, 3, 4 or 5, Z represents a hydrogen atom; and 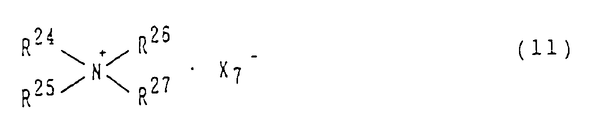 wherein R²⁴ represents a branched alkyl group having 8 to 28 carbon atoms in total; R²⁵ represents a straight-chain alkyl or alkenyl group having 8 to 22 carbon atoms; R²⁶ and R²⁷ each represent a hydrogen atom or an alkyl group having 1 to 4 carbon atoms; and X₇⁻ represents a halogen ion or an organic anion; provided that R²⁶ and R²⁷ do not represent a hydrogen atom simultaneously.

6. The hair conditioner composition of claim 5, wherein component (B) is at least one compound selected from quaternary ammonium salts represented by formula (1).

7. The hair conditioner composition of claim 5 or 6 wherein said halogen ion is selected from the group consisting of a chloride ion, a bromide ion and an iodide ion.

8. The hair conditioner composition of any one of claims 5 to 7 wherein said organic anion is selected from the group consisting of a methosulfate ion, an ethosulfate ion, a methophosphate ion and an ethophosphate ion.

9. A method for imparting gloss to hair which comprises applying to hair a hair conditioner composition comprising (A) a fatty acid having a straight-chain acyl group containing 18 to 28 carbon atoms, and (B) a cationic surfactant, wherein a weight ratio of component (A) to component (B) is from 1.5/1 to 4/1.

## Patentansprüche

1. Haar-Konditionierzusammensetzung, umfassend:
(A) eine Fettsäure mit einer geradkettigen Acylgruppe mit 18 bis 28 Kohlenstoffatomen und
(B) ein kationisches oberflächenaktives Mittel,
worin das Gewichtsverhältnis der Komponente (A) zur Komponente (B) 1,5/1 bis 4/1 beträgt.

2. Haar-Konditionierzusammensetzung gemäß Anspruch 1, worin der pH-Wert der genannten Zusammensetzung 2 bis 7 beträgt.

3. Haar-Konditionierzusammensetzung gemäß Anspruch 1 oder 2, worin Komponente (A) in einer Menge von 1,0 bis 15,0 Gew.% und Komponente (B) in einer Menge von 0,25 bis 7,5 Gew.% vorhanden sind.

4. Haar-Konditionierzusammensetzung gemäß einem der Ansprüche 1 bis 3, worin die genannte Komponente (A) Behensäure ist.

5. Haar-Konditionierzusammensetzung gemäß einem der Ansprüche 1 bis 4, worin Komponente (B) mindestens ein quaternäres Ammoniumsalz ist, ausgewählt aus der Gruppe, bestehend aus denjenigen der Formeln (1), (2), (7) und (11): 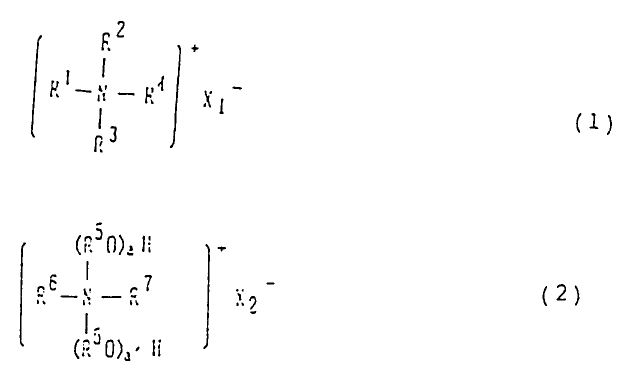 worin mindestens einer der Reste R¹, R², R³ und R⁴ eine Alkyl- oder Alkenylgruppe mit insgesamt 8 bis 28 Kohlenstoffatomen, die mit einem Substituent substituiert sein kann, ausgewählt aus einer Alkoxy-, Alkenyloxy-, Alkanoylamino- und aus einer Alkenoylaminogruppe, und die weiteren Reste eine Benzyl- oder eine Alkyl- oder Hydroxyalkylgruppe mit 1 bis 5 Kohlenstoffatomen, R⁵ eine Alkylengruppe mit 2 oder 3 Kohlenstoffatomen, X₁⁻ und X₂⁻ jeweils ein Halogenion oder ein organisches Anion, a und a' jeweils eine ganze Zahl von 1 bis 20 und mindestens einer der Reste R⁶ und R⁷ eine Alkyl- oder Alkenylgruppe mit insgesamt 8 bis 28 Kohlenstoffatomen, die mit einem Substituent substituiert sein kann, ausgewählt aus einer Alkoxy-, Alkenyloxy-, Alkanoylamino- und aus einer Alkenoylaminogruppe, und der andere Rest eine Benzyl- oder eine Alkyl- oder Hydroxyalkylgruppe mit 1 bis 5 Kohlenstoffatomen darstellen; 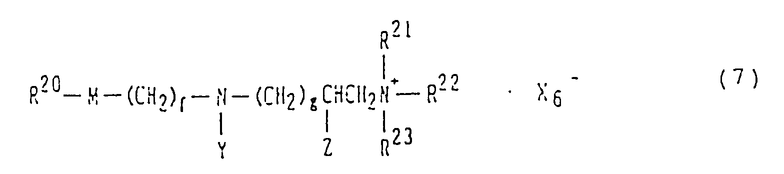 worin R²⁰ eine geradkettige oder verzweigte Alkyl- oder Alkenylgruppe mit 7 bis 21 Kohlenstoffatomen, R²¹, R²² und R²³ jeweils eine Alkyl- oder Hydroxyalkylgruppe mit 1 bis 4 Kohlenstoffatomen, M -CONG- (worin G ein Wasserstoffatom oder eine Alkyl- oder Hydroxyalkylgruppe mit 1 bis 3 Kohlenstoffatomen darstellt, mit der Maßgabe, dass die Carbonylgruppe davon an R²⁰ gebunden ist), -O- oder -COO-(mit der Maßgabe, dass die Carbonylgruppe davon an R²⁰ gebunden ist), X₆⁻ ein Halogenion oder ein organisches Anion, f 2 oder 3, g 0 oder eine ganze Zahl von 1 bis 5, Z ein Wasserstoffatom oder eine Hydroxylgruppe und Y ein Wasserstoffatom, eine Alkyl- oder Hydroxyalkylgruppe mit 1 bis 3 Kohlenstoffatomen oder eine Gruppe der Formel (8) darstellen: 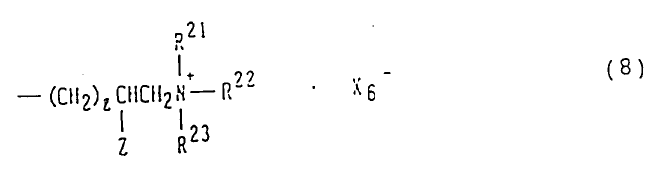 worin R²¹, R²², R²³, X₆⁻, Z und g wie oben definiert sind, mit der Maßgabe, dass G und Y nicht gleichzeitig eine Alkyl- oder Hydroxyalkylgruppe mit 1 bis 3 Kohlenstoffatomen darstellen, und dass, wenn g 0, 2, 3, 4 oder 5 ist, Z ein Wasserstoffatom darstellt; und 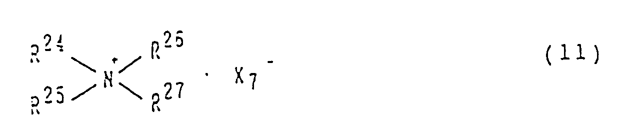 worin R²⁴ eine verzweigte Alkylgruppe mit insgesamt 8 bis 28 Kohlenstoffatomen, R²⁵ eine geradkettige Alkyl- oder Alkenylgruppe mit 8 bis 22 Kohlenstoffatomen, R²⁶ und R²⁷ jeweils ein Wasserstoffatom oder eine Alkylgruppe mit 1 bis 4 Kohlenstoffatomen und X₇⁻ ein Halogenion oder ein organisches Anion darstellen, mit der Massgabe, dass R²⁶ und R²⁷ nicht gleichzeitig ein Wasserstoffatom darstellen.

6. Haar-Konditionierzusammensetzung gemäß Anspruch 5, worin Komponente (B) mindestens eine Verbindung ist, ausgewählt aus quaternären Ammoniumsalzen der Formel (1).

7. Haar-Konditionierzusammensetzung gemäß Anspruch 5 oder 6, worin das genannte Halogenion aus der Gruppe ausgewählt ist, bestehend aus einem Chlorid-, Bromid- und einem Jodidion.

8. Haar-Konditionierzusammensetzung gemäß einem der Ansprüche 5 bis 7, worin das genannte organische Anion aus der Gruppe ausgewählt ist, bestehend aus einem Methosulfat-, Ethosulfat-, Methophosphat- und einem Ethophdsphation.

9. Verfahren zur Übertragung von Glanz auf das Haar, wobei man auf das Haar eine Haar-Konditionierzusammensetzung aufbringt, umfassend (A) eine Fettsäure mit einer geradkettigen Acylgruppe mit 8 bis 28 Kohlenstoffatomen und (B) ein kationisches oberflächenaktives Mittel, worin das Gewichtsverhältnis von Komponente (A) zur Komponente (B) 1,5/1 bis 4/1 beträgt.

## Revendications

1. Composition de conditionnement de cheveux comprenant :
(A) un acide gras ayant un groupe acyle à chaîne droite contenant 18 à 28 atomes de carbone, et
(B) un tensioactif cationique
caractérisée en ce que le rapport en poids du composant (A) au composant (B) est de 1,5/1 à 4/1.

2. Composition de conditionnement de cheveux selon la revendication 1, caractérisée en ce que ladite composition a un pH de 2 et 7.

3. Composition de conditionnement de cheveux selon les revendications 1 et 2, caractérisée en ce que le composant (A) est présent en une quantité de 1,0 à 15,0% en poids et le composant (B) est présent en une quantité de 0,25 à 7,5% en poids.

4. Composition de conditionnement de cheveux selon l'une quelconque des revendications 1 à 3, caractérisée en ce que ledit composant (A) est l'acide béhénique.

5. Composition de conditionnement de cheveux selon l'une quelconque des revendications 1 à 4, caractérisée en ce que le composant (B) est au moins un sel d'ammonium quaternaire choisi dans le groupe constitué par ceux représentés par les formules (1), (2), (7), et (11) : 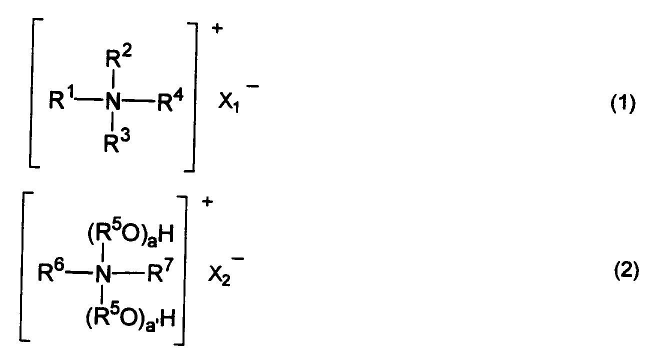 formules dans lesquelles au moins un de R¹, R², R³, et R⁴ représente un groupe alkyle ou alcényle ayant 8 à 28 atomes de carbone au total, qui peut être substitué par un substituant choisi parmi les groupes alcoxy, alcényloxy, alcanoylamino et alcénoylamino, et le ou les autres représentent un groupe benzyle ou un groupe alkyle ou hydroxyalkyle ayant 1 à 5 atomes de carbone ; R⁵ représente un groupe alkylène ayant 2 ou 3 atomes de carbone ; X₁⁻ et X₂⁻ représentent chacun un ion halogène ou un anion organique ; a et a' représentent chacun un entier de 1 à 20 ; et au moins un de R⁶ et R⁷ représente un groupe alkyle ou alcényle ayant 8 à 28 atomes de carbone au total, qui peut être substitué par un substituant choisi parmi les groupes alcoxy, alcényloxy, alcanoylamino et alcénoylamino, et l'autre représente un groupe benzyle ou un groupe alkyle ou hydroxyalkyle ayant 1 à 5 atomes de carbone ; 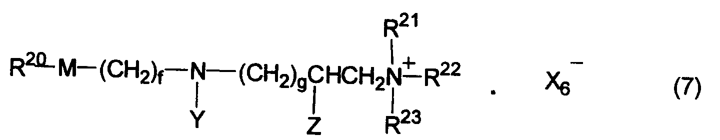 formule dans laquelle R²⁰ représente un groupe alkyle ou alcényle à chaîne droite ou ramifiée ayant 7 à 21 atomes de carbone; R²¹, R²², et R²³ représentent chacun un groupe alkyle ou hydroxyalkyle ayant 1 à 4 atomes de carbone ; M représente -CONG- (où G représente un atome d'hydrogène ou un groupe alkyle ou hydroxyalkyle ayant 1 à 3 atomes de carbone, sous réserve que le groupe carbonyle de celui-ci soit lié à R²⁰), -O- ou -COO- (sous réserve que le groupe carbonyle de celui-ci soit lié à R²⁰); X₆⁻ représente un ion halogène ou un anion organique ; f représente 2 ou 3; g représente 0 ou un entier de 1 à 5 ; Z représente un atome d'hydrogène ou un groupe hydroxyle; et Y représente un atome d'hydrogène, un groupe alkyle ou hydroxyalkyle ayant 1 à 3 atomes de carbone, ou un groupe représenté par la formule (8) : 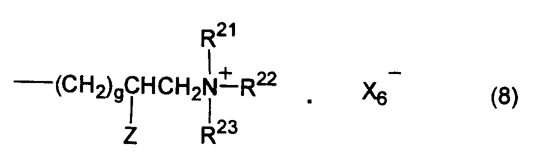 formule dans laquelle R²¹, R²², R²³, X₆⁻, Z, et g sont tels que définis ci-dessus ; sous réserve que G et Y ne représentent pas simultanément un groupe alkyle ou hydroxyalkyle ayant 1 à 3 atomes de carbone et que quand g vaut 0, 1, 2, 3, 4 ou 5, Z représente un atome d'hydrogène ; et 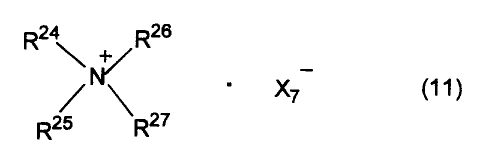 formule dans laquelle R²⁴ représente un groupe alkyle ramifié ayant 8 à 28 atomes de carbone au total; R²⁵ représente un groupe alkyle ou alcényle à chaîne droite ou ramifiée ayant 8 à 22 atomes; R²⁶ et R²⁷ représentent chacun un atome d'hydrogène ou un groupe alkyle ayant 1 à 4 atomes de carbone ; et X₇⁻ représente un ion halogène ou un anion organique ; sous réserve que R²⁶ et R²⁷ ne représentent pas simultanément un atome d 'hydrogène.

6. Composition de conditionnement de cheveux selon la revendication 5, caractérisée en ce que le composant (B) est au moins un composé choisi parmi les sels d'ammonium quaternaire représentés par la formule (1).

7. Composition de conditionnement de cheveux selon la revendication 5 ou 6, caractérisée en ce que ledit ion halogène est choisi dans le groupe constitué par un ion chlorure, un ion bromure et un ion iodure.

8. Composition de conditionnement de cheveux selon l'une quelconque des revendications 5 à 7, caractérisée en ce que ledit anion organique est choisi dans le groupe constitué par un ion méthosulfate, un ion éthosulfate, un ion méthophosphate et un ion éthophosphate.

9. Procédé pour conférer une brillance aux cheveux qui comprend l'application aux cheveux d'une composition de conditionnement de cheveux comprenant (A) un acide gras ayant un groupe acyle à chaîne droite contenant 18 à 28 atomes de carbone, et (B) un tensioactif cationique, caractérisé en ce que le rapport en poids du composant (A) au composant (B) est de 1,5/1 à 4/1.
